# EUROPEAN PATENT APPLICATION

(11) **EP 1 609 413 A1**
(43) Date of publication of application: **28.12.2005**
(21) Application number: 05013263.8
(22) Date of filing: 20.06.2005
(51) Int. Cl.: A61B 5/022

(54) **Sphygmomanometer clamping device**

(30) Priority: 23.06.2004 JP 2004184879
(71) Applicant: Omron Healthcare Co., Ltd., Kyoto 615-0084 (JP)
(72) Inventor: Kishimoto, Hiroshi, Omron Healthcare Co.Ltd, Kyoto-shi Kyoto 615-0084 (JP); Sano, Yoshihiko , Omron Healthcare Co.Ltd, Kyoto-shi Kyoto 615-0084 (JP); Karo, Hiromichi, Omron Healthcare Co.Ltd, Kyoto-shi Kyoto 615-0084 (JP); Miyata, Kiichiro, Omron Healthcare Co.Ltd, Kyoto-shi Kyoto 615-0084 (JP); Eda, Kenji, Omron Healthcare Co.Ltd, Kyoto-shi Kyoto 615-0084 (JP); Tanaka, Takahide, Omron Healthcare Co.Ltd, Kyoto-shi Kyoto 615-0084 (JP)
(74) Representative: Kilian, Helmut

(57) **Abstract**

A sphygmomanometer clamping device (2) includes an actuator (21) that includes an Electroactive Polymer Artificial Muscle (EPAM) expandable and contractible when a voltage is applied to the EPAM and electrodes provided to apply the voltage to a elastomer or a polymer, and a curler (22) deformed to clamp a part of a living body according to an expansion or a contraction of the EPAM.

## Description

### Background of the invention

### Field of the invention

The present invention relates to a clamping device used for a sphygmomanometer.

### Description of the Related Art

During measurement of blood pressure, it is necessary to wind an bladder around a part of a human body, e.g., an upper arm and constrain and fix the bladder so as to accurately detect a arterial pressure pulse wave generated by the pressing an artery of the human body.

At this time, it is necessary to constrain and fix the upper arm according to a size of the upper arm. Due to this, after the upper arm is inserted into a cuff (an arm band for pressing a wrist or an arm), the cuff is automatically wound around the upper arm according to the size of the upper arm and the air is fed to the bladder within the cuff, thereby constraining and pressing the upper arm.

As a device of this type, a floating mechanism that includes a winding clutch that winds and transmit a winding wire rope around a pulley connected to a motor so as to wind a cuff including an bladder, a rewinding clutch, and a lock clutch that stops looseness of an arm band during pressurization is disclosed in Japanese Utility Model Application Laid-Open No. 2-135003.

In addition, a blood pressure measurement arm band winding device that includes a flexible arm band formed into a cylinder, a tape and a winding roller for winding the flexible armband, and a motor that rotates the winding roller, and that is configured so that the arm band is fixed by rotating the winding roller is disclosed in Japanese Patent Application Laid-Open No. 10-314123.

Further, an automatic blood pressure measurement arm band winding device that includes a movable member movably stored in a housing chamber, a bellows coupled to the movable member, and a pump that feeds a compressed air to the bellows, and that is configured so that the bellows is extended by the compressed air, thereby moving the movable member and winding a cuff around a living body is disclosed in Japanese Patent Application Laid-Open No. 7-124128.

The conventional techniques have, however, the following disadvantages. As a mechanism section included in a winding unit that fixedly winds the cuff or flexible arm band around a part of the human body, a driving transmission section such as the wire rope, the pulley, the clutch, or the roller as well as the motor that drives the driving transmission section is required. This disadvantageously increases the number of parts, with the result that the device is made complicated, large, and heavy.

Furthermore, if the number of parts increases and the device is complicated, noise and current consumption are increased.

### Summary of the Invention

The present invention has been achieved in view of the conventional techniques. It is an object of the present invention to provide a sphygmomanometer clamping device having a simple configuration and an electronic sphygmomanometer using the same.

According to the present invention, in order to achieve said object there is provided a sphygmomanometer clamping device comprising: an actuator including an elastomer or a polymer expandable or contractible when a voltage is applied to the elastomer or polymer, and electrodes provided to apply the voltage to the elastomer or polymer; and a fixture deformed to clamp a part of a living body according to an expansion or a contraction of the elastomer or polymer.

As the elastomer or polymer expandable or contractible when a voltage is applied thereto, a matter referred to as an dielectric elastomer or electrostrictive polymer (e.g., high strain plastic having electric field response such as silicone resin, acrylic resin, or polyurethane) can be used. An electroactive polymer artificial muscle ("EPAM") is most preferably used as the expandable or contractible elastomer or polymer. In addition, the shape of the fixture may preferably be generally ring-shape.

With this constitution, the sphygmomanometer clamping device having a simple configuration without the need of a component such as a motor or clutch so as to deform the fixture for clamping a part of the living body can be realized.

In the sphygmomanometer clamping device, if a high voltage is applied to the actuator for long time, a capacitor effect appears. Thanks to this, during measurement of the human body (clamping thereof), power necessary to hold a state in which the human body is clamped and constrained can be saved.

Preferably, the sphygmomanometer clamping device further comprises a fluid bag provided inside of the fixture, and pressurized during a blood pressure measurement, wherein the fixture clamps the part of the living body through the fluid bag.

In the sphygmomanometer clamping device, the fixture exhibits flexibility or elasticity, whereby it is possible to deform the fixture and to perform the deformation operation repeatedly even if a force generated by the actuator is low.

In the sphygmomanometer clamping device, the fixture is formed into a ring by connecting at least two components, the components being plate-like or R-like components. It is thereby possible to deal with measurement of regions having different circumferential lengths by changing the number of components. The R shape is a curved surface and not a flat surface. The curved surface is preferably a curved surface, e.g., a circular arc-like curved surface, according to the shape of the measurement target region.

In the sphygmomanometer clamping device, the actuator is arrangedbetween the components adjacent each other, and a plurality of actuators are provided. It is thereby possible to greatly change the circumferential length of the clamping device.

In the sphygmomanometer clamping device, each of the components includes a projection (protrusion) guided by a slide portion provided on the component adjacent to the component, the component moving relatively to the adjacent component according to an expansion or a contraction of the actuator. It is thereby possible to deform the clamping device without detaching the components from one another.

In the sphygmomanometer clamping device, it is preferable that each of the components includes a plurality of projections guided by a plurality of slide portions provided on the component adjacent to the component, the components moving relatively to the adjacent component according to an expansion or a contraction of the actuator, and the projections differing in amounts by which the projections move relative to the slide portions.

With this constitution, if a part of the living body to be measured, which is a region, such as the upper arm or the wrist, having different diameters between the side on which the region is inserted into the fixture and the side on which the region protrudes from the fixture, is to be measured, the slide amount of each slide portion is determined according to the shape of the upper arm. As a result, a clamping force of the clamping device can be made uniform throughout the measurement region, and accurate blood pressure measurement can be ensured.

In the sphygmomanometer clamping device, the fixture may be deformed according to a shape of the part of the living body during clamping of the living body, and an inside diameter of the fixture on a side on which the living body is inserted into the fixture may be larger than an inside diameter of the fixture on a side on which the living body protrudes from the fixture.

The sphygmomanometer clamping device may further comprise deformation amount amplification unit to amplify a deformation amount of the actuator, and for deforming the fixture.

The sphygmomanometer clamping device further comprises an elastic member that restores the actuator from a deformation, whereby the deformation from the clamping state to the unclamping state can be promptly performed.

In the sphygmomanometer clamping device, an electrode is provided on a junction portion that connects the actuator to the fixture. It is thereby possible to reduce the size of the clamping device.

In the sphygmomanometer clamping device, the actuator includes conductive expansion materials provided on both surfaces of the polymer that can expand and contract, as electrodes, respectively. It is thereby possible to stably apply the voltage to the polymer films or the like even if the actuator is deformed.

In the sphygmomanometer clamping device, the actuator includes a first elastomer or polymer contracting in a circumferential direction of the fixture when the voltage is applied thereto, and a second elastomer or polymer expanding in the circumferential direction of the fixture when the voltage is applied thereto. By controlling voltages applied to the first and second elastomers or polymers, respectively, it is possible to clamp and unclamp the actuator even if the spring portion is not provided. Thus, the number of components can be reduced, and the clamping device can be made small in size and light in weight.

Further, the sphygmomanometer clamping device is preferably used with an electronic sphygmomanometer comprising: a pump that pressurizes the fluid bag; a pressure sensor that detects an internal air pressure of the fluid bag; and arithmetic unit to execute a processing for the blood pressure measurement based on the detected internal air pressure. It is thereby possible to realize the electronic sphygmomanometer small in size and light in weight.

The present invention can realize the sphygmomanometer clamping device having the simple configuration. In addition, the present invention can realize the electronic sphygmomanometer small in size and light in weight.

### Brief description of the drawings

Figs. 1A and 1B are cross-sectional views of a clamping device according to a first embodiment;
Fig. 2 is a perspective view of the clamping device according to the first embodiment;
Fig. 3 is a block diagram that depicts a hardware configuration of an electronic sphygmomanometer;
Fig. 4 is a flowchart that depicts a basic operation of the electronic sphygmomanometer;
Figs. 5A and 5B are schematic views that depict a state of deformation when a voltage is applied to an EPAM according to the embodiment;
Figs. 6A and 6B are cross-sectional views that depict a clamping operation of a clamping device according to a second embodiment;
Figs. 7A and 7B are perspective view that depicts the clamping operation of the clamping device according to the second embodiment;
Figs. 8A and 8B are perspective views that depict a clamping operation of a clamping device according to a third embodiment;
Figs. 9A and 9B are perspective views that depict a clamping operation of a clamping device according to a fourth embodiment;
Figs. 10A and 10B are perspective views that depict a clamping operation of a clamping device according to a fifth embodiment;
Figs. 11A and 11B are cross-sectional views of an EPAM according to the fifth embodiment;
Fig. 12 is a cross-sectional view that depicts a state of a junction portion;
Fig. 13 is a perspective view that depicts a configuration of a clamping device according to a sixth embodiment;
Fig. 14 is a perspective view that depicts a configuration of a clamping device according to a seventh embodiment; and
Figs. 15A and 15B are schematic views for explaining a structure of the EPAM according to the first embodiment.

### Detailed description of the preferred embodiments

Most preferred embodiments of the present invention will be described hereinafter in detail with reference to the drawings and the embodiments. It should be noted, however, that sizes, materials, shapes, functions, relative positions, etc. of constituent members described in the embodiment are not intended to limit the scope of the present invention unless specified otherwise. Further, sizes, materials, shapes, functions, etc. of the members described once in the following explanation are the same as those explained initially unless specified otherwise.

### (CONFIGURATION OF SPHYGMOMANOMETER)

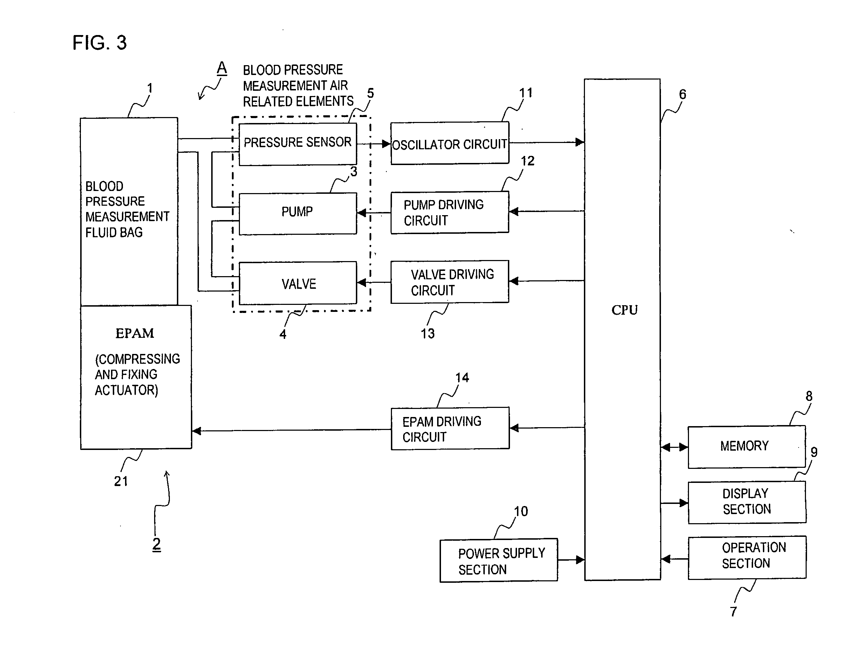

Fig. 3 is a block diagram that depicts a hardware configuration of an electronic sphygmomanometer to which the present invention can be suitably applied.

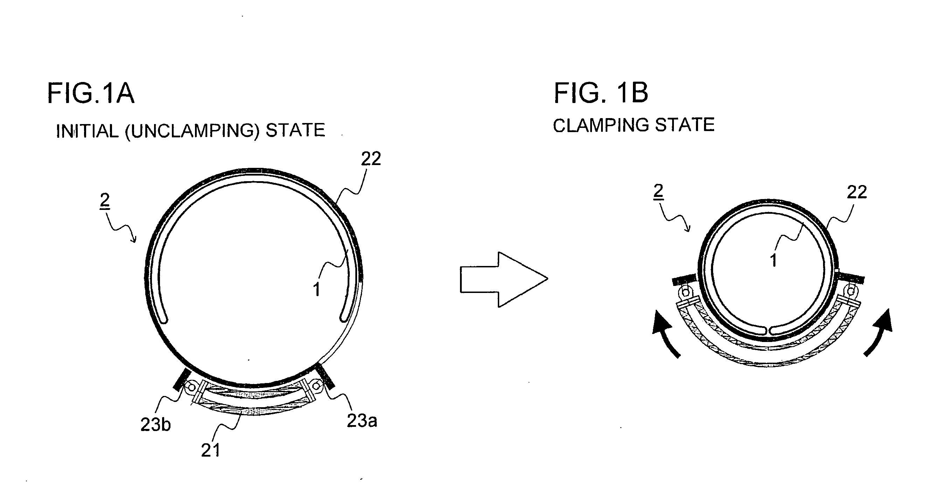

An electronic sphygmomanometer A includes a clamping device 2 that includes a fluid bag 1 wound around an upper arm during blood pressure measurement, and a compressing and fixing actuator 21 that surrounds the fluid bag 1 and that thereby compresses and fixes the fluid bag 1, and a curler 22 (see Fig. 1) serving as a fixture (hereinafter referred to as a "clamping device"), a pump 3 that feeds a fluid to the fluid bag 1 filled with the fluid such as the air and pressurizes the fluid bag 1, a valve 4 that discharges the fluid within the fluid bag 1, a pressure sensor 5 that detects an internal air pressure of the fluid bag 1, a central processing unit (CPU) 6 serving as arithmetic unit to execute a processing for blood pressure measurement according to a program included in the CPU 6 based on the detected internal air pressure, an operation section 7 that makes settings during the measurement and that starts the measurement, a memory 8 that stores setting data, arithmetic data, measurement results, etc., a display section 9 that displays a setting state, the measurement result, and the like, and a power supply section 10 that supplies power to the respective constituent sections.

Further, the CPU 6 detects an internal pressure of the fluid bag 1 based on a signal output from the pressure sensor 5 and converted by an oscillator circuit 11. The CPU 6 controls a pump driving circuit 12 to drive the pump 3 to increase the internal pressure of the fluid bag 1 if it is necessary to pressurize the fluid bag 1. The CPU 6 controls a valve driving circuit 13 to open the valve 4 to reduce the internal pressure of the fluid bag 1 if it is necessary to reduce the internal pressure of the fluid bag 1.

The CPU 6 also controls a driving circuit 14 to drive the clamping device 2 so as to clamp the upper arm through the fluid bag 1.

As the clamping device 2, a device using an Electroactive Polymer Artificial Muscle ("EPAM") to be described later is preferable. In the following embodiments, instances in which the EPAM is used in the actuator 21 will be described.

### (BASIC OPERATION OF SPHYGMOMANOMETER)

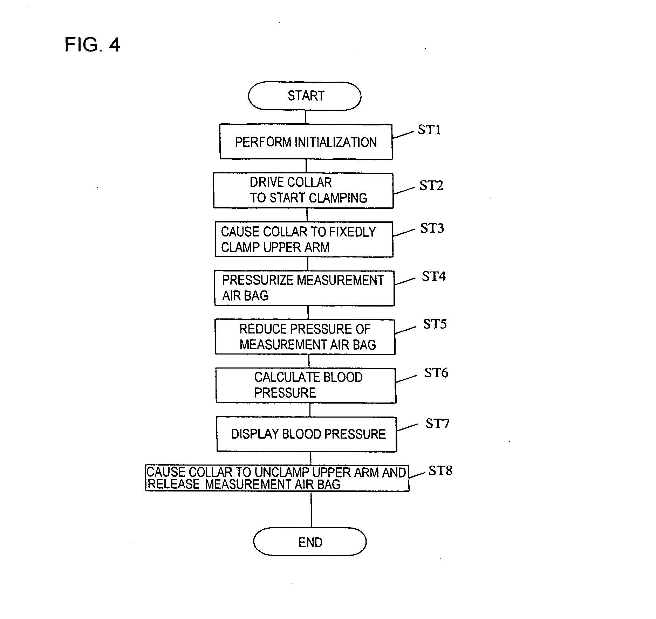

Fig. 4 is a flowchart that depicts a basic operation of the electronic sphygmomanometer to which the present invention can be suitably applied.

When a power of the electronic sphygmomanometer A is turned on and the electronic sphygmomanometer A starts operating, initialization for resetting respective setting states of the electronic sphygmomanometer A to initial states is performed (in a step ST1).

The electronic sphygmomanometer A drives the clamping device 2, whereby the curler serving as the fixture that constitutes a part of the clamping device 2 starts clamping the upper arm (in a step ST2). Thereafter, the curler is fixed to the upper arm while clamping the upper arm to some extent (in a step ST3).

The fluid bag 1 arranged between the upper arm and the curler fixed to the upper arm is pressurized up to a predetermined pressure by the pump 3. The signal detected by the pressure sensor 5 and indicating a pressure change of the fluid bag 1 is transmitted to the CPU 6 through the oscillator circuit 11, and a blood pressure measurement is started based on the signal (in a step ST4).

The internal pressure of the fluid bag 1 is then gradually reduced by opening the valve 4 (in a step ST5). The CPU 6 calculates a systolic blood pressure, a diastolic blood pressure, and a pulse frequency (in a step ST6). The calculated blood pressure and the like are displayed on the display section 9 (in a step ST7).

When the measurement is finished, the driving circuit 14 drives the clamping device 2 to unclamp the curler. In addition, the air within the fluid bag 1 that has compressed the upper arm is discharged from the valve 4, whereby the upper arm is unclamped (in a step ST8), thus finishing one measurement operation cycle.

A configuration of the preferred blood pressure measurement clamping device according to the present invention will be described. An instance of measuring the blood pressure of the upper arm of a human body will be described below. However, the blood pressure measurement clamping device is also applicable to living bodies other than the human body, and a measurement target region may be a wrist or an ankle that is a part of the living body.

### [FIRST EMBODIMENT]

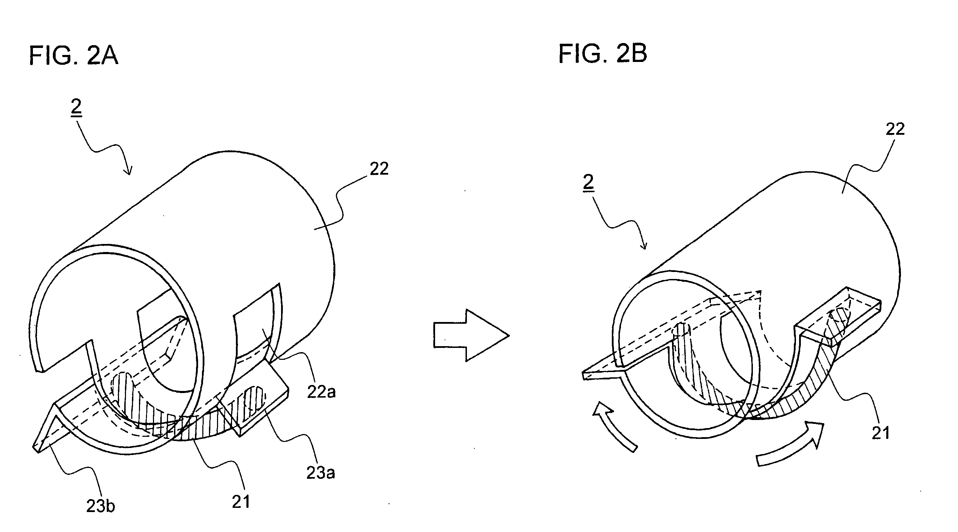

Figs. 1A and 1B are cross-sectional views that depict a clamping operation of a clamping device according to a first embodiment. Fig. 2 is a perspective view that depicts the clamping operation of the clamping device according to the first embodiment.

The clamping device 2 shown in Figs. 1A and 1B includes the actuator 21 that includes an EPAM which can expand or contract when a voltage is applied thereto and electrodes (not shown) for applying the voltage to the EPAM, the curler 22 that is a generally ring-shaped fixture deformed to clamp a part of the living body according to the expansion or contraction of the EPAM and serving to fixedly clamp the fluid bag 1 to the upper arm, and junction portions 23a and 23b that connect ends of the actuator 21 to the curler 22.

The fluid bag 1 is provided within the curler 22 and pressurized during the blood pressure measurement. The curler 22 clamps a part of the living body, e.g., the upper arm through the fluid bag 1.

As shown in Fig. 2, the curler 22 is of a cylindrical shape configured so that a plate-like elastic member having either flexibility or elasticity is wound by about one turn. The curler 22 includes the junction portion 23a provided on one end of a plate while being bent in a radial direction, and the junction portion 23b provided on the other end of the plate while being bent in the radial direction. The actuator 21 is attached between the junction portions 23a and 23b with the junction portion 23a projecting outward from an opening 22a formed in a part of the plate. It is thereby possible to deform the curler 22 even if a force generated by the actuator 21 is low and to perform the deformation operation repeatedly.

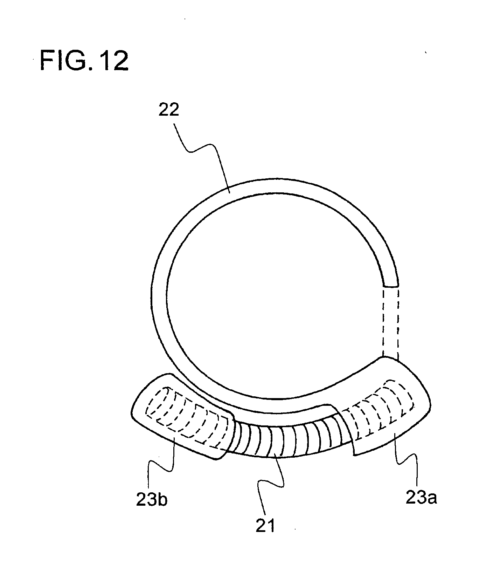

It suffices that the junction portions 23a and 23b that connect the actuator 21 to the curler 22 are configured so that the actuator 21 is not detached during the operation of the clamping device 2. For example, the junction portions 23a and 23b may be cylindrical junction portions provided with holes into which the both ends of the actuator 21 are inserted as shown in Fig. 12.

Further, by providing each of the junction portions 23a and 23b with an electrode, a voltage can be easily applied to the actuator 21.

For example, if a clamping voltage is applied to both surfaces of the expandable and contractible polymer film by electrode films having elastic property (e.g., conductive expansion materials having carbon particles dispersed to elastic members), a distance between the electrodes is reduced and the polymer films expand laterally. By using this property for the clamping device 2, therefore, the clamping device 2 can clamp the upper arm with a simple configuration without using a motor. In addition, by using the conductive expansion materials for the electrodes, a stable voltage can be applied to the polymer films and the like even if the actuator 21 is deformed.

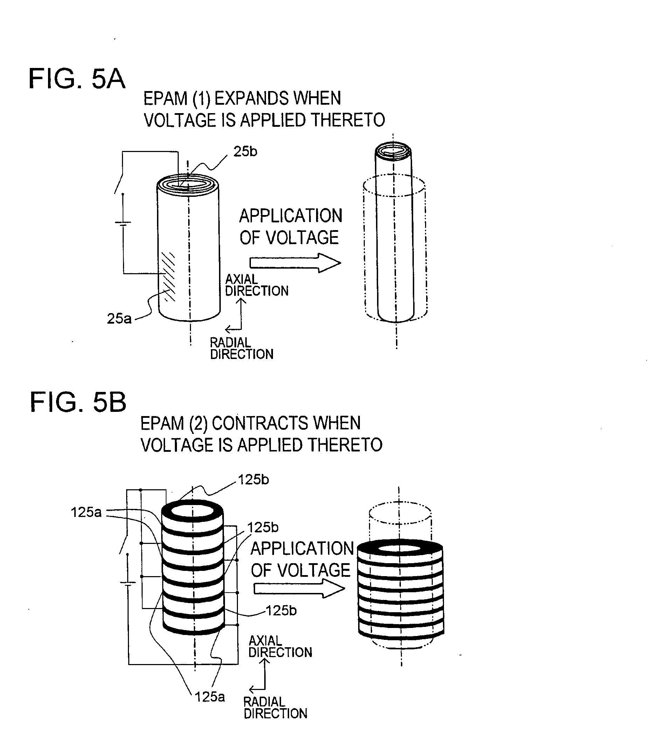

Figs. 5A and 5B are schematic views that depict a state of deformation when a voltage is applied to the actuator according to this embodiment.

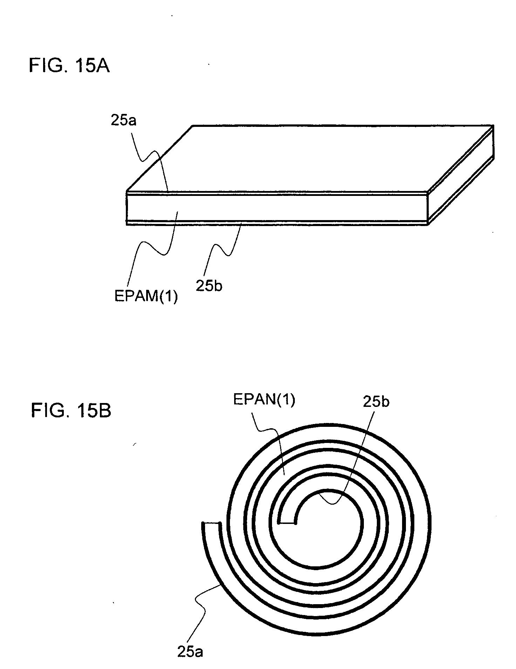

The actuator 21 according to the first embodiment is configured so that a film-like EPAM (1) having expansion electrodes 25a and 25b provided on both surfaces, respectively, as shown in Fig. 15A, is wound into a roll by one turn or a plurality of turns as shown in Fig. 15B. An insulator, not shown, is wound into a roll together with the EPAM (1) to isolate the adjacent electrodes from each other when the EPAM (1) is wound. As shown in Fig. 5A, a voltage V is applied between the electrodes 25a and 25b, whereby the actuator 21 contracts in a radial direction and expands in an axial direction. Due to this, the actuator 21 expands along a circumferential direction of the curler 22 and the distance between the junction portions 23a and 23b is increased, and an inside diameter of the curler 22 is reduced. The upper arm can be, therefore, clamped.

By using this actuator 21, the clamping device 2 according to the first embodiment can deform the curler 22 from an initial (unclamping) state (Fig. 1A) to a clamping state (Fig. 1B) without using a complicated mechanism such as a motor or a clutch.

If the application of the voltage is stopped, then the deformed actuator 21 returns to its original shape by the elastic property (elastic force) of the curler 22, and the curler 22 returns from the clamping state to the initial state. It is also effective to provide the actuator 21 with a spring portion so as to intensify a restoring force of the curler 22 itself.

The clamping device 2 according to the first embodiment can, therefore, perform the clamping operation superior in response to the motor with the simple configuration without the need of the complicated mechanism such as the motor or clutch as shown in the conventional techniques. It is thereby possible to contribute to reductions in the size and weight of the sphygmomanometer.

Further, since a motor driving sound is not produced, noise generated while the actuator is driven can be reduced. Besides, since there is no need to apply a current for driving the motor, the power consumption of the entire sphygmomanometer can be suppressed.

### [Second Embodiment]

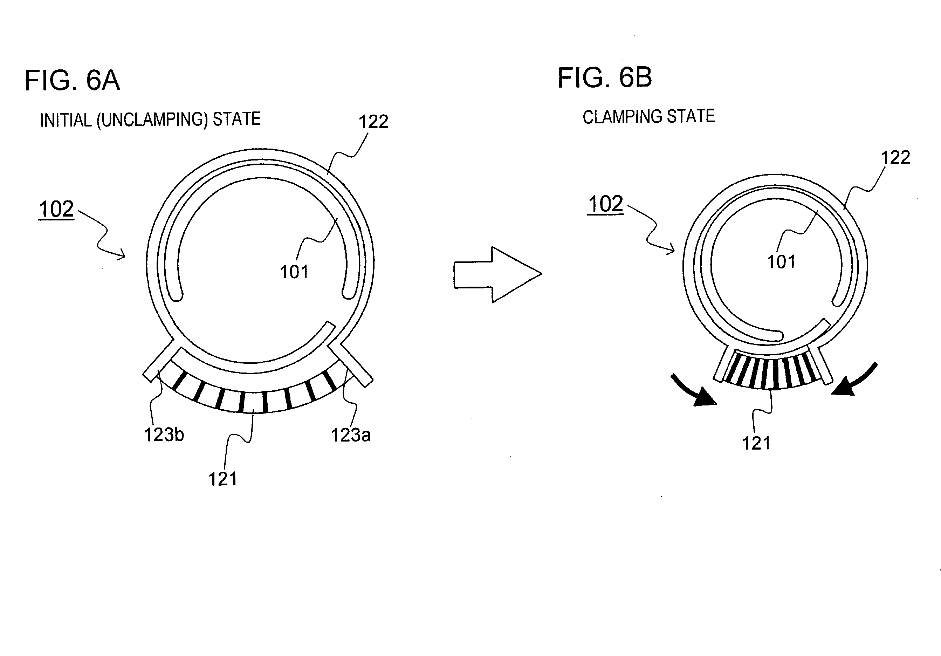

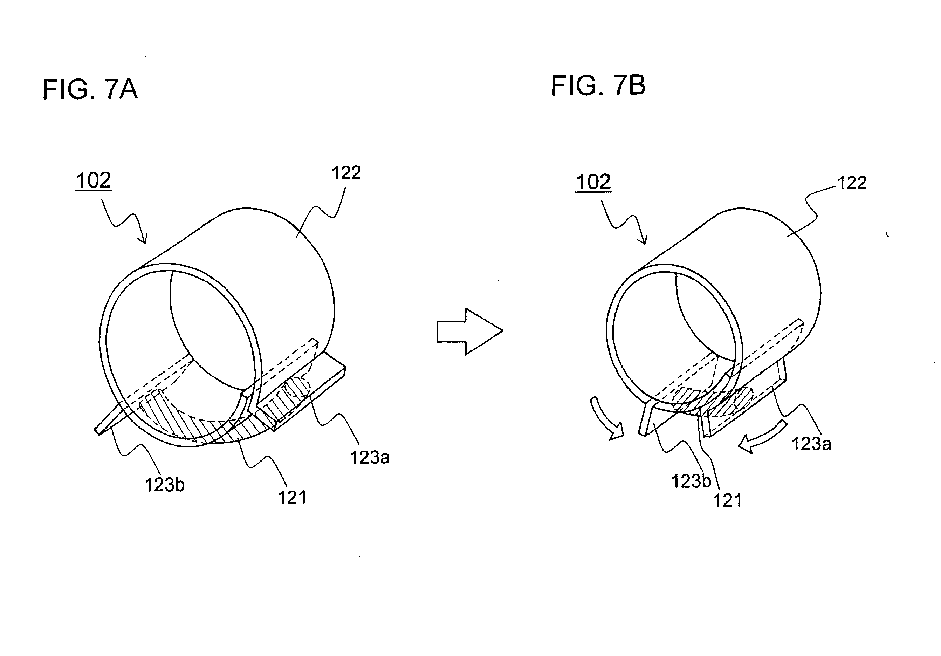

Figs. 6A and 6B are cross-sectional views that depict a clamping operation of a clamping device according to a second embodiment. Figs. 7A and 7B are perspective views that depict the clamping operation of the clamping device according to the second embodiment.

A clamping device 102 shown in Figs. 6A and 6B includes an actuator 121 that includes an EPAM which can expand or contract when a voltage is applied thereto and electrodes (not shown) for applying the voltage to the EPAM, a curler 122 that is a generally ring-shaped fixture deformed to clamp a part of the living body according to the expansion or contraction of the EPAM and fixedly clamping a fluid bag 101 to the upper arm, junction portions 123a and 123b that connect ends of the actuator 121 to the curler 122, and electrodes (not shown) arranged to apply the voltage to the actuator 121.

As shown in Figs. 7A and 7B, the curler 122 is of a cylindrical shape configured so that one end of a plate-like elastic member having either flexibility or elasticity is bent and that the elastic member is wound by about one turn so as to overlap both ends thereof with each other. A junction portion 123a bent in a radial direction is provided on one end of the curler 122 so that the actuator 121 can be attached to the curler 122. In addition, the actuator 121 is heldbetween a plate-like junction portion 123b provided on an outer peripheral portion of the curler 122 to protrude in the radial direction and the junction portion 123a.

As shown in Fig. 5B, the actuator 121 according to the second embodiment is configured so that electrodes 125a and 125b are provided on axially both ends of an EPAM (2), respectively. Further, in this embodiment, a plurality of electrodes 125a and 125b are alternately provided on the EPAM (2). By applying a voltage V between the electrodes 125a and 125b, the actuator 121 contracts in the axial direction and expands in the radial direction. Due to this, the actuator 121 contracts along a circumferential direction of the curler 122 and the distance between the junction portions 123a and 123b is reduced. Therefore, an inside diameter of the curler 122 is reduced and the upper arm can be clamped.

By using this actuator 121, the clamping device 102 according to the second embodiment can deform the curler 122 from an initial (unclamping) state (Fig. 6A) to a clamping state (Fig. 6B) without using a complicated mechanism such as a motor or a clutch.

If the application of the voltage is stopped, then the deformed actuator 121 returns to its original shape by the elastic property (elastic force) of the curler 122, and the curler 122 returns from the clamping state to the initial state. A spring may be provided between the junction portions 123a and 123b so as to use an elastic force of the spring if it is necessary to do so.

The clamping device 102 according to the second embodiment can, therefore, perform the clamping operation superior in response to the motor with the simple configuration without the need of the complicated mechanism such as the motor or clutch as shown in the conventional techniques. It is thereby possible to contribute to reductions in the size and weight of the sphygmomanometer.

Further, since a motor driving sound is not produced, noise generated while the actuator is driven can be reduced. Besides, since there is no need to apply a current for driving the motor, the power consumption of the entire sphygmomanometer can be suppressed.

### [Third Embodiment]

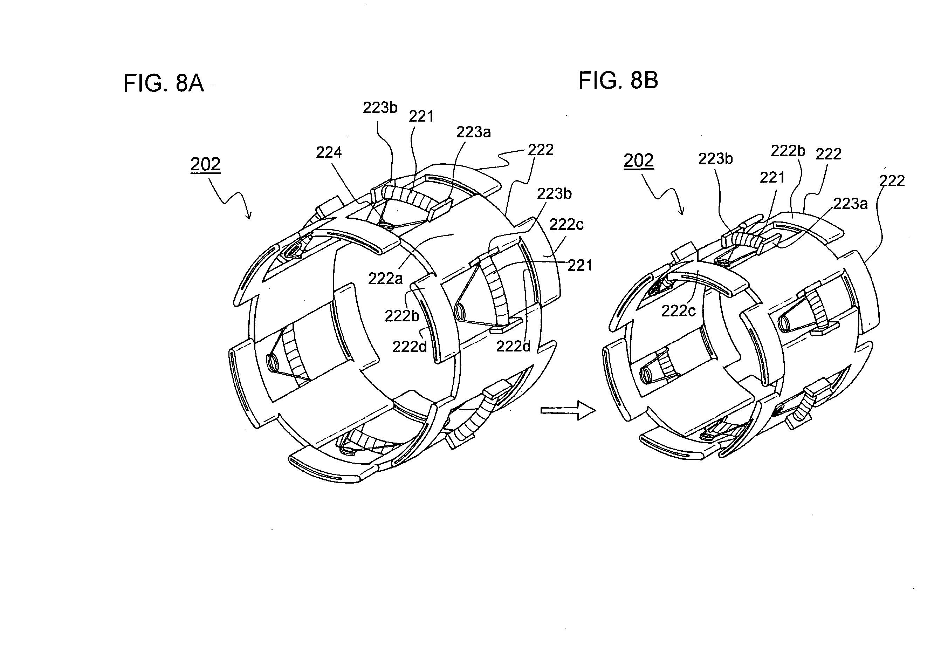

Figs. 8A and 8B are perspective views that depict a clamping operation of a clamping device according to a third embodiment.

A clamping device 202 shown in Figs. 8A and 8B includes a plurality of divided curlers 222, a plurality of actuators 221 contracting in an electric field direction and expanding in a direction perpendicular to the electric field direction when a voltage is applied thereto, junction portions 223a and 223b that connect ends of the actuators 221 to the respective divided curlers 222, spring portions 224 serving as expansion and contraction portions that hold an inside diameter of each curler to such an extent as to be able to insert the upper arm into the curlers 222 while no voltage is applied thereto, and electrodes (not shown) arranged on the junction portions between the actuators 221 and the divided curlers 222 to apply a voltage to the actuator 221.

Each of the divided curlers 222 includes a circular arc-shaped main body portion 222a provided with the junction portions 223a and 223b, slide portions 222b and 222c slidable relative to the adjacent main body portion 222a, and a slide groove 222d into which a projection (not shown) provided on the adjacent main body portion 222a is inserted. By connecting at least two or more plate-like or R-like divided curlers 222, the ring-like fixture is constituted.

The slide portions 222b and 222c are provided on both ends of a side parallel to the axial direction of the main body portion 222a, respectively. The divided curlers 222 may be formed by connecting a plurality of components integrally or by integrall molding.

Since each curler 222 includes the projection guided by the slide portions 222b and 222c provided on the adjacent divided curler 222, each divided curler 222 can be moved relatively to the adjacent divided curler 222 according to expansion or contraction of the actuator 221.

Further, the actuator 221 is attached between the junction portion 223a of the divided curler 222 and the j unction portion 223b of the adj acent divided curler 222. In addition, the spring portion 224 that generates pressure in such a direction that the entire curler is widened during clamping is provided between the divided curlers 222 corresponding to the actuator 221.

The actuator 221 according to the third embodiment can employ the EPAM (2) employed in the second embodiment and shown in Fig. 5B. For example, by applying a voltage between electrodes (not shown) provided on the junction portions 223a and 223b, each cylindrical actuator 221 contracts in an axial direction of the cylinder and expands in a radial direction of the cylinder. Due to this, the each actuator 221 contracts along a circumferential direction of the divided curler 222 and the distance between the junction portions 223a and 223b is reduced. Therefore, an inside diameter of the clamping device 202 configured to connect the divided curlers 222 can be reduced and the upper arm can be clamped.

By using these actuators 221, the clamping device 202 according to the third embodiment can deform each divided curler 222 from an initial (unclamping) state (Fig. 8A) to a clamping state (Fig. 8B) without using a complicated mechanism such as a motor or a clutch.

If the application of the voltage is stopped, then each deformed actuator 221 returns to its original shape by the elastic force of the spring portion 224, and each divided curler 222 returns from the clamping state to the initial state.

The clamping device 202 according to the third embodiment can, therefore, perform the clamping operation superior in response to the motor with the simple configuration without the need of the complicated mechanism such as the motor or clutch as shown in the conventional techniques. It is thereby possible to contribute to reductions in the size and weight of the sphygmomanometer.

Further, since a motor driving sound is not produced, noise generated while the actuator is driven can be reduced. Besides, since there is no need to apply a current for driving the motor, the power consumption of the entire sphygmomanometer can be suppressed.

Moreover, each divided curler 222 includes a plurality of projections (not shown) guided by a plurality of slide portions 222b and 222c provided on the adjacent divided curler 222. In addition, the divided curler 222 is configured to be moved relatively to the adjacent divided curler 222 correspond to expansion and contraction of the actuator 221, and to be able to set amounts by which the respective projections move relatively to each of the slide portions 222b and 222c differently from one another.

The clamping device 202 including a plurality of divided curlers 222 is configured to be deformable according to a shape of the upper arm when clamping the upper arm. In addition, the clamping device 202 is configured to be deformable so that an inside diameter thereof on the side on which the upper arm is inserted into the curlers is larger than an inside diameter thereof on the side on which the upper arm protrudes from the curlers.

In other words, the two slide portions 222b and 222c sliding relatively to the adjacent divided curler 222 are provided per divided curler. Due to this, if a region, e.g., the upper arm, having different diameters between the side on which the upper arm is inserted into the curlers and the side on which the upper arm protrudes from the curlers, the slide amount of each slide portion is set according to the shape of the upper arm. Specifically, the region closer to the hand in the upper arm measurement target region has a smaller diameter, so that the slide amount of each slide portion is increased accordingly. On the other hand, the region closer to the shoulder has a larger diameter, so that the slide amount of each slide portion is decreased accordingly. As a result, a clamping force of the clamping device 202 can be made uniform throughout the measurement region, and accurate blood pressure measurement can be ensured.

Moreover, by employing the divided curlers 222, it is possible to deal with measurement of regions having different circumferential lengths by changing the number of divided curlers 222.

Furthermore, by using a plurality of divided curlers 222 and arranging each actuator between the divided curlers, the circumferential length of the clamping device 202 can be greatly changed. It is, therefore, possible to increase the inside diameter of the clamping device 202 before measurement (in the unclamping state) . This can facilitate inserting the wrist or upper arm of the measured person, and lessen a sense of oppression given to the measured person.

### [FOURTH EMBODIMENT]

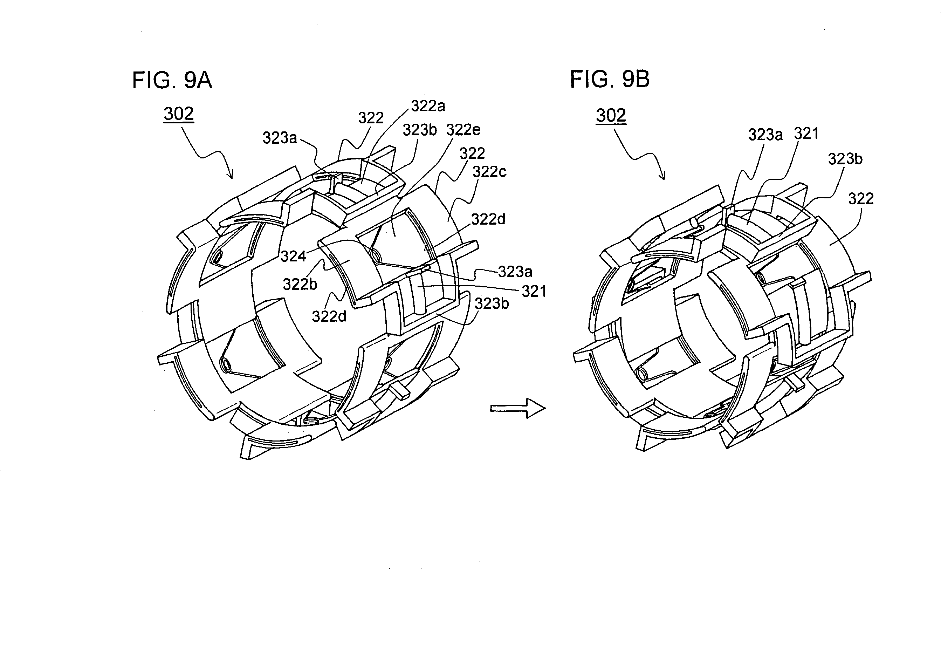

Figs. 9A and 9B are perspective views that depict a clamping operation of a clamping device according to a fourth embodiment.

A clamping device 302 shown in Figs. 9A and 9B includes a plurality of divided curlers 322, a plurality of actuators 321 contracting in an electric field direction and expanding in a direction perpendicular to the electric field direction when a voltage is applied thereto, junction portions 323a and 323b that connect (j oin) ends of the actuators 321 to the respective divided curlers 322, spring portions 324 serving as expansion and contraction portions each of which holds an inside diameter of each curler 322 to such an extent as to be able to insert the upper arm into the curler 322 while no voltage is applied, and electrodes (not shown) arranged on the junction portions between the actuators 321 and the divided curlers 322 to apply a voltage to the actuator 321.

Each of the divided curlers 322 includes a circular arc-shaped main body portion 322a provided with a junction portion 323a, slide portions 322b ad 322c slidable relative to the adj acent main body portion 322a, a slide groove 322d into which a projection (not shown) provided on the adjacent main body portion 322a is inserted, an opening 322e surrounded by the main body portion 322a and the slide portions 322b and 322c, and a junction portion 323b provided on an opposite side to the main body portion 322a with the opening 322e held between the main body portion 322a and the junction portion 323b.

One end of each of the slide portions 322b and 322c is connected to both ends of a side parallel to the axial direction of the main body portion 322a. The other end thereof is connected to the junction portion 323b. The divided curlers 322 may be formed by connecting a plurality of components integrally or by integrall molding.

Each actuator 321 is provided between the junction portion 323a of the divided curler 322 and the junction portion 323b of the adjacent divided curler 322. In addition, the spring portion 324 that generates pressure in a direction in which the entire curlers 322 extend during clamping is provided in each opening 322e correspond to the actuator 321.

The actuator 321 according to the fourth embodiment can employ the EPAM (1) employed in the first embodiment and shown in Fig. 5A. For example, by applying a voltage between electrodes (not shown) provided on the junction portions 323a and 323b, each cylindrical actuator 321 contracts in a radial direction of the cylinder and expands in an axial direction of the cylinder. Due to this, the each actuator 321 expands along a circumferential direction of the divided curler 322 and the distance between the junction portions 323a and 323b is increased. Therefore, an inside diameter of the clamping device 302 configured to connect the divided curlers 322 can be reduced and the upper arm can be thereby clamped.

By using these actuators 321, the clamping device 302 according to the fourth embodiment can deform each divided curler 322 from an initial (unclamping) state (Fig. 9A) to a clamping state (Fig. 9B) without using a complicated mechanism such as a motor or a clutch.

If the application of the voltage is stopped, then each deformed actuator 321 returns to its original shape by the elastic force of the spring portion 324, and each divided curler 322 returns from the clamping state to the initial state.

The clamping device 302 according to the fourth embodiment can, therefore, perform the clamping operation superior in response to the motor with the simple configuration without the need of the complicated mechanism such as the motor or clutch as shown in the conventional techniques. It is thereby possible to contribute to reductions in the size and weight of the sphygmomanometer.

Further, since a motor driving sound is not produced, noise generated while the actuator is driven can be reduced. Besides, since there is no need to apply a current for driving the motor, the power consumption of the entire sphygmomanometer can be suppressed.

Moreover, by employing the divided curlers 322, the clamping device 302 according to the fourth embodiment can deal with measurement of regions having different circumferential lengths by changing the number of divided curlers 322.

Furthermore, by using a plurality of divided curlers 322 and arranging each EPAM between the divided curlers, the circumferential length of each actuator 321 can be greatly changed. It is, therefore, possible to increase the inside diameter of the actuator 321 before measurement (in the unclamping state). This can facilitate inserting the wrist or upper arm of the measured person, and lessen a sense of oppression given to the measured person.

### [FIFTH EMBODIMENT]

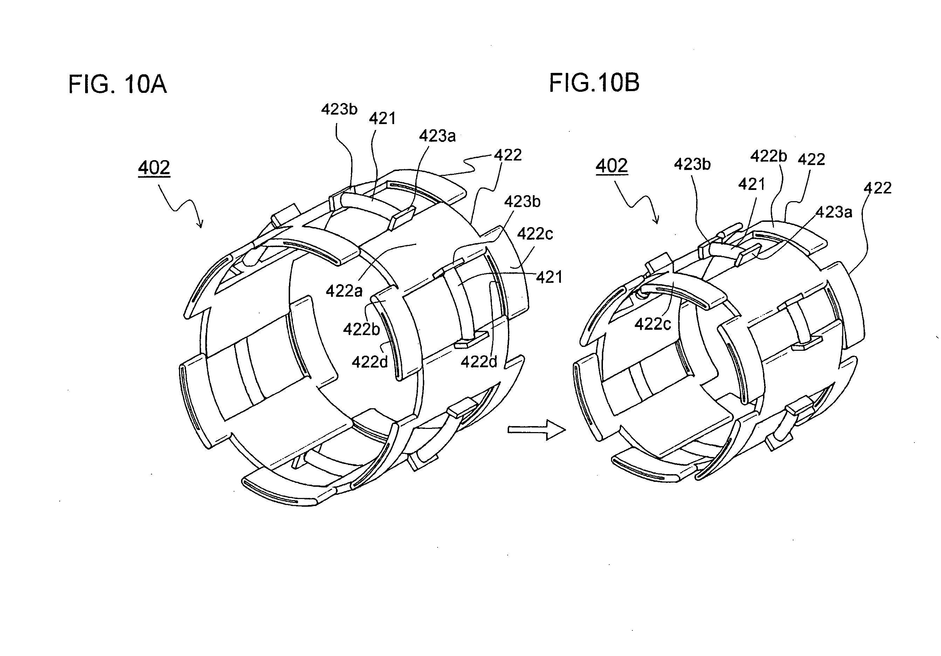

Figs. 10A and 10B are perspective views that depict a clamping operation of a clamping device according to a fifth embodiment.

A clamping device 402 shown in Figs. 10A and 10B are similar in configuration to the clamping device 202 shown in Figs. 8A and 8B except for the following respects. In the clamping device 402, the actuators 221 having the same voltage application direction are changed to actuators 421 each of which includes two types of EPAMs having different voltage application directions. In addition, the spring portions 224 are not provided.

Namely, each actuator 421 according to the fifth embodiment includes a first EPAM that contracts in a circumferential direction of the curler when a voltage is applied thereto and a second EPAM that expands in the circumferential direction of the curler when a voltage is applied thereto.

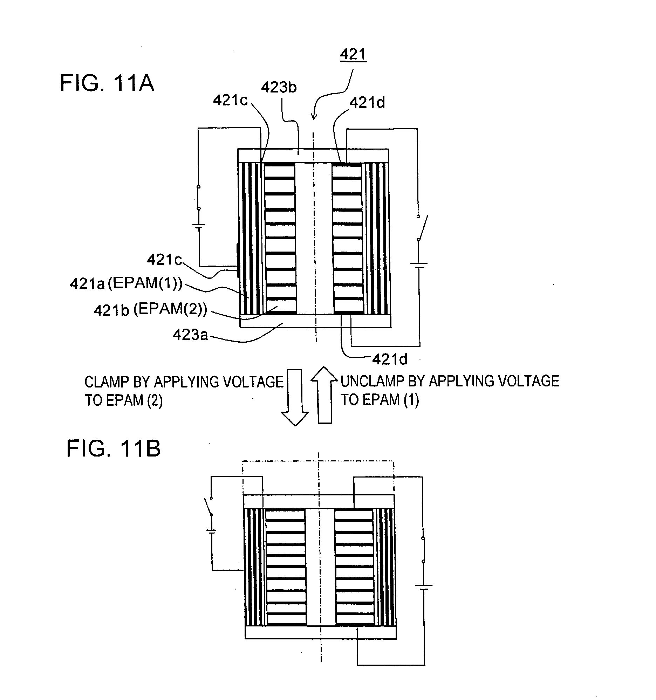

The clamping device 402 shown in Fig. 10 includes a plurality of divided curlers 222, a plurality of actuators 421 each including one of two types of EPAM 421a and 421b (see Fig. 11) contracting in an electric field direction or expanding in a direction perpendicular to the electric field direction when a voltage is applied to the actuator, junction portions 423a and 423b that connect ends of each actuator 421 to the divided curlers 422, and electrodes (not shown) provided on the junction portion between each of the EPAMs 421a and 421b and the divided curler 422 to apply a voltage to the respective EPAMs 421a and 421b.

Figs. 11A and 11B are cross-sectional views of the EPAM according to the fifth embodiment configured by a pair of electrodes 421c and 421d provided to apply voltages to the EPAMs 421a and 421b, respectively.

The same constituent elements of the actuator according to the fifth embodiment as those of the actuator according to the third embodiment will not be described but different constituent elements, functions, and advantages thereof will be described.

The actuator 421 according to the fifth embodiment can employ the EPAMs (1) and (2) shown in Figs. 5A and 5B employed in the first and second embodiments. In the fifth embodiment, the actuator 421 is of a cylindrical shape configured so that the EPAM 421a (EPAM (1)) wound into a roll as shown in Fig. 5A is arranged on an outer side of the actuator 421, and so that the cylindrical EPAM 421b (EPAM (2)) shown in Fig. 5B is arranged on an inner side.

By applying no voltage between the pair of electrodes 421c but applying the voltage between the pair of electrodes 421d (see Figs. 11A and 11B), the cylindrical EPAM 421b contracts in an axial direction of the cylinder and expands in a radial direction of the cylinder. Due to this, the overall actuator 421 contracts along a circumferential direction of the divided curler 422 and the distance between the junction portions 423a and 423b is decreased. Due to this, an inside diameter of the clamping device 402 configured to connect the divided curlers 422 is decreased, and it is thereby possible to clamp the upper arm.

By applying no voltage between the pair of electrodes 421d but applying the voltage between the pair of electrodes 421c (see Figs. 11A and 11B), the cylindrical EPAM 421a contracts in a radial direction of the cylinder and expands in an axial direction of the cylinder. Due to this, the overall actuator 421 expands along the circumferential direction of the divided curler 422 and the distance between the junction portions 423a and 423b is increased. An inside diameter of the clamping device 402 configured to connect the divided curlers 422 is increased, and it is thereby possible to unclamp the upper arm.

The clamping device 402 according to the fifth embodiment can, therefore, perform the clamping operation superior in response to the motor with the simple configuration without the need of the complicated mechanism such as the motor or clutch as shown in the conventional techniques. It is thereby possible to contribute to reductions in the size and weight of the sphygmomanometer.

Further, since a motor driving sound is not produced, noise generated while the actuator is driven can be reduced. Besides, since there is no need to apply a current for driving the motor, the power consumption of the entire sphygmomanometer can be suppressed.

Moreover, the two types of EPAMs having different voltage application directions are provided and the voltages applied to the respective types of EPAMs are controlled. It is thereby possible to perform the clamping and unclamping operations on the actuators even if the spring portions are not provided. Thus, the number of components can be reduced and the size and weight of the device can be reduced.

### [SIXTH EMBODIMENT]

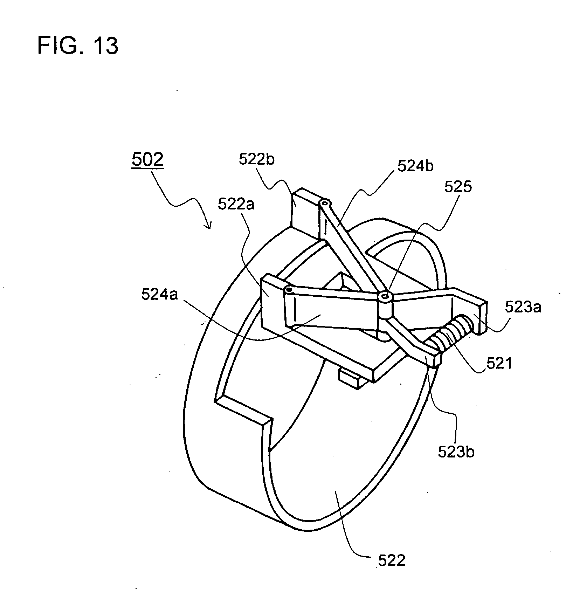

Fig. 13 is a perspective view that depicts a schematic configuration of a clamping device according to a sixth embodiment.

A clamping device 502 shown in Fig. 13 includes a curler 522 that fixedly clamps an bladder (air bag), not shown, to the upper arm, an actuator 521 that contracts in an electric field direction and expands in a direction perpendicular to the electric field direction when a voltage is applied thereto, junction portions 523a and 523b that connect ends of the actuator 521 to the curler 522, and electrodes (not shown) arranged to apply the voltage to the actuator 521.

The curler 522 is of a cylindrical shape configured so that one end of a plate-like elastic member is bent and that the elastic member is wound by about one turn so as to overlap both ends thereof with each other. One end 522a of the curler 522 is connected to the junction portion 523a by a coupling portion 524a whereas the other end 522b thereof is connected to the junction portion 523b by a coupling portion 524b. The coupling portions 524a and 524b rotatably cross each other at a fulcrum 525, and move around the fulcrum 525 according to expansion or contraction of the actuator 521 held between the junction portions 523a and 523b.

The actuator 521 according to the sixth embodiment can employ the EPAMs shown in Figs. 5A and 5B, for example, by appropriately selecting one of the EPAMs. By applying a voltage between electrodes (not shown) provided on both ends of the actuator 521, respectively, the actuator 521 contracts in the axial direction and expands in the radial direction. Due to this, the actuator 521 expands along a circumferential direction of the curler 522 and the distance between the junction portions 523a and 523b is increased. Therefore, an inside diameter of the curler 522 is reduced and the upper arm can be clamped.

Namely, the clamping device 502 according to the sixth embodiment is configured to connect the junction portions to the ends of the curler so as to be able to utilize the principle of a lever as deformation amount amplification unit to amplify a deformation amount of the actuator 521 and for deforming the curler 522, and to enable the coupling portions to relatively rotate about the fulcrum.

The clamping device 502 according to the sixth embodiment can, therefore, deform the curler 522 from an initial (unclamping) state to a clamping state without using a complicated mechanism such as a motor or a clutch by using the above-stated configuration.

Further, if the application of the voltage is stopped, then the deformed actuator 521 returns to its original shape by the elastic property (elastic force) of the curler 522, and the curler 522 returns from the clamping state to the initial state.

Moreover, the clamping device 502 according to the sixth embodiment can perform the clamping operation superior in response to the motor with the simple configuration without the need of the complicated mechanism such as the motor or clutch as shown in the conventional techniques. It is thereby possible to contribute to reductions in the size and weight of the sphygmomanometer.

Further, since a motor driving sound is not produced, noise generated while the actuator is driven can be reduced. Besides, since there is no need to apply a current for driving the motor, the power consumption of the entire sphygmomanometer can be suppressed.

### [SEVENTH EMBODIMENT]

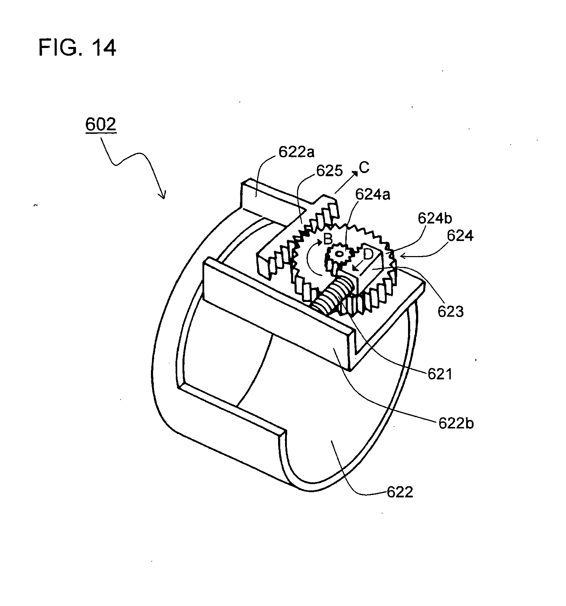

Fig. 14 is a perspective view that depicts a schematic configuration of a clamping device according to a seventh embodiment.

A clamping device 602 shown in Fig. 14 includes a curler 622 that fixedly clamps an bladder, not shown, to the upper arm, an actuator 621 that contracts in an electric field direction and expands in a direction perpendicular to the electric field direction when a voltage is applied thereto, a rack 623 fixed to one end of the actuator 621, double gears 624 arranged so that one of the gears 624 is engaged with the rack 623, a rack 625 engaged with the other one of the double gears 624, and electrodes (not shown), arranged to apply the voltage to the actuator 621.

The curler 622 is of a cylindrical shape configured so that one end of a plate-like elastic member is bent and that the elastic member is wound by about one turn so as to overlap both ends thereof with each other. The rack 625 is attached to one end 622a of the curler 622. The other end of the actuator 621 is connected to the other end 622b of the curler 622.

The actuator 621 according to the seventh embodiment can employ the EPAMs shown in Figs. 5A and 5B, for example, by appropriately selecting one of the EPAMs. By applying a voltage between electrodes (not shown) provided on both ends of the actuator 621, respectively, the actuator 621 contracts in the axial direction and expands in the radial direction.

Due to this, the rack 623 attached to the actuator 621 moves in an arrow D direction, a small-diameter gear 624a engaged with the rack 623 rotates, and a large-diameter gear 624b also rotates in an arrow B direction. By causing the rack 625 engaged with the gear 624b to move in an arrow C direction, the distance between the both ends 622a and 622b is increased. Therefore, an inside diameter of the curler 622 is reduced and the upper arm can be clamped.

Namely, the clamping device 602 according to the seventh embodiment employs a combination of gears and racks as deformation amount amplification unit to amplify a deformation amount of the actuator 621 and for deforming the curler 622.

The clamping device 602 according to the seventh embodiment can, therefore, deform the curler 622 from an initial (unclamping) state to a clamping state without using a complicated mechanism such as a motor or a clutch by using the above-stated configuration and by appropriately selecting a gear ration based on characteristics (e.g., an expansion ratio and an operating pressure) of the actuator 621.

Further, if the application of the voltage is stopped, then the deformed actuator 621 returns to its original shape by the elastic property (elastic force) of the curler 622, and the curler 622 returns from the clamping state to the initial state.

Moreover, the clamping device 602 according to the seventh embodiment can perform the clamping operation superior in response to the motor with the simple configuration without the need of the complicated mechanism such as the motor or clutch as shown in the conventional techniques. It is thereby possible to contribute to reductions in the size and weight of the sphygmomanometer.

Further, since a motor driving sound is not produced, noise generated while the actuator is driven can be reduced. Besides, since there is no need to apply a current for driving the motor, the power consumption of the entire sphygmomanometer can be suppressed.

## Claims

1. A sphygmomanometer clamping device comprising:
an actuator including an elastomer or a polymer expandable or contractible when a voltage is applied to the elastomer or polymer, and electrodes provided to apply the voltage to the elastomer or polymer; and
a fixture deformed to clamp a part of a living body according to an expansion or a contraction of the elastomer or polymer.

2. A sphygmomanometer clamping device according to claim 1, further comprising:
a fluid bag provided inside of the fixture, and pressurized during a blood pressure measurement, wherein
the fixture clamps the part of the living body through the fluid bag.

3. A sphygmomanometer clamping device according to claim 1 or 2, wherein
the fixture exhibits flexibility or elasticity.

4. A sphygmomanometer clamping device according to any one of claims 1 to 3, wherein
the fixture is formed into a ring by connecting at least two components, the components being plate-like or R-like components.

5. A sphygmomanometer clamping device according to claim 4, wherein
the actuator is arranged between the components adjacent each other, and
a plurality of actuators are provided.

6. A sphygmomanometer clamping device according to claim 4 or 5, wherein
each of the components includes a projection guided by a slide portion provided on the component adjacent to the component, the components moving relatively to the adjacent component according to an expansion or a contraction of the actuator.

7. A sphygmomanometer clamping device according to claim 4 or 5, wherein
each of the components includes a plurality of projections guided by a plurality of slide portions provided on the component adjacent to the component, the components moving relatively to the adjacent component according to an expansion or a contraction of the actuator, and the projections differing in amounts by which the projections move relative to the slide portions.

8. A sphygmomanometer clamping device according to any one of claims 1 to 5, wherein
the fixture is deformed according to a shape of the part of the living body during clamping of the living body, an inside diameter of the fixture on a side on which the living body is inserted into the fixture being larger than an inside diameter of the fixture on a side on which the living body protrudes from the fixture.

9. A sphygmomanometer clamping device according to any one of claims 1 to 8, further comprising:
deformation amount amplification unit to amplify a deformation amount of the actuator, and for deforming the fixture.

10. A sphygmomanometer clamping device according to any one of claims 1 to 9, further comprising:
an elastic member that restores the actuator from a deformation.

11. A sphygmomanometer clamping device according to any one of claims 1 to 10, wherein
an electrode is provided on a junction portion that connects the actuator to the fixture.

12. A sphygmomanometer clamping device according to any one of claims 1 to 11, wherein
the actuator includes conductive expansion materials provided on both surfaces of the elastomer or polymer that can expand and contract, as electrodes, respectively.

13. A sphygmomanometer clamping device according to any one of claims 1 to 12, wherein
the actuator includes a first elastomer or polymer contracting in a circumferential direction of the fixture when the voltage is applied thereto, and a second elastomer or polymer expanding in the circumferential direction of the fixture when the voltage is applied thereto.

14. An electronic sphygmomanometer comprising:
the sphygmomanometer clamping device according to any one of claims 2 to 13;
a pump that pressurizes the fluid bag;
a pressure sensor that detects an internal air pressure of the fluid bag; and
arithmetic unit to execute a processing for the blood pressure measurement based on the detected internal air pressure.
